# EUROPEAN PATENT APPLICATION

(11) **EP 3 338 854 A1**
(43) Date of publication of application: **27.06.2018**
(21) Application number: 16837056.7
(22) Date of filing: 10.08.2016
(51) Int. Cl.: A61M 39/06, A61M 39/04

(54) **VALVE BODY FOR MEDICAL DEVICE, MEDICAL DEVICE, AND METHOD FOR MANUFACTURING VALVE BODY FOR MEDICAL DEVICE**

(30) Priority: 17.08.2015 JP 2015160398
(71) Applicant: Goodman Co., Ltd., Aichi 460-0008 (JP)
(72) Inventor: HIWA, Hideto, Seto-shi Aichi 489-0976 (JP); MASUDA, Jun, Seto-shi Aichi 489-0976 (JP)
(74) Representative: Epping - Hermann - Fischer
(86) International application number: PCT/JP2016/073557
(87) International publication number: WO 2017/030066

(57) **Abstract**

A hemostasis valve (5) is mounted on a Y-connector used to introduce an introduction member into a living organism. The hemostasis valve (5) is provided with: a base (50) having a first surface (51A) and a second surface (52A), which oppose each other; and a first slit and a second slit, which are provided in the base (50). The first slit extends from the first surface (51A) toward the second surface (52A) by a first slit length (T3) that is shorter than a base length (T0), which is the length between the first surface (51A) and the second surface (52A). The second slit extends from the second surface (52A) toward the first surface (51A) by a second slit length (T4) that is shorter than the base length (T0). The first slit and the second slit are connected at a connection section. In a single state, the gap of the second slit is smaller than that of the first slit.

## Description

### Technical Field

The present invention relates to a valve body for a medical device configured to be assembled to a medical device that is used to introduce an introduction member, such as a catheter, into a living organism, to the medical device, and to a method for manufacturing the valve body for the medical device.

### Background art

A Y connector is known as an example of a medical device used when carrying out treatment inside a blood vessel using a catheter. The Y connector is provided with a valve body that is used to suppress bleeding from the blood vessel. Patent Literature 1 discloses a Y connector that is provided with a hemostasis valve. The Y connector is provided with a main body unit, and the hemostasis valve. An insertion hole, through which a guide wire is inserted, is provided in the main body unit. The hemostasis valve is assembled to the main body unit so as to block the insertion hole. A slit is formed in the hemostasis valve. The hemostasis valve has a very close fit with the guide wire that passes through the slit. The hemostasis valve suppresses a flow of blood from a gap between the slit and the guide wire.

### Citation List

### Patent Literature

Patent Literature 1: Japanese Laid-Open Patent Publication No. 2014-39771

### Summary of Invention

In order to appropriately suppress the flow of blood, it is preferable for the gap of the slit of the hemostasis valve to be small. This is because, the smaller the gap of the slit, the stronger the close fit of the hemostasis valve with the guide wire, the catheter or the like (hereinafter referred to as an "introduction member") that is inserted through the insertion hole. On the other hand, when the hemostasis valve has a strong close fit with the introduction member, the introduction member has difficulty moving with respect to the hemostasis valve. In this case, operability when a user introduces the introduction member into the blood vessel sometimes deteriorates.

An object of the present invention is to provide a valve body for a medical device capable of suppressing a flow of blood when a user introduces an introduction member into a living organism using the medical device and also capable of improving operability of the introduction member, and to provide the medical device and a method for manufacturing the valve body for the medical device.

A valve body for a medical device according to a first aspect of the present invention is a valve body for a medical device that is configured to be assembled to the medical device used to introduce an introduction member into a living organism. The valve body for the medical device is characterized by including: a base including a first surface and a second surface that oppose each other; and slits provided in the base, wherein the slits include a first slit and a second slit, the first slit extending from the first surface toward the second surface by a first length, the first length being shorter than a base length that is a length between the first surface and the second surface, the second slit extending from the second surface toward the first surface by a second length that is shorter than the base length, and at least a part of each of the first slit and the second slit are connected at a connecting section, when the introduction member is introduced into the living organism, the introduction member passes through the connecting section and moves from the first surface side toward the second surface side, and in a single state in which the valve body is not assembled with the medical device, a gap of the second slit is smaller than a gap of the first slit.

The valve body for the medical device includes the first slit and the second slit that have the different gap sizes in the single state. The introduction member is introduced into the living organism by moving the connecting section between the first slit and the second slit from the first surface side to the second surface side. The base has a strong close fit with the introduction member on the second surface side, on which the second slit having the relatively small gap is provided. Thus, the valve body for the medical device may appropriately suppress the blood inside the living organism from flowing from a gap between the base and the introduction member. The valve body for the medical device may weaken the close fit between the base and the introduction member on the first surface side, on which the first slit having the relatively large gap is provided. As a result, the valve body for the medical device may improve operability when a user moves the introduction member from the first surface side toward the second surface side.

In the first aspect, in the single state, the gap of the second slit may be 0. In this case, there is an even closer fit between the base and the introduction member on the second surface side on which the second slit is provided. Thus, the valve body for the medical device may even more appropriately suppress the blood inside the living organism from flowing from the gap between the base and the introduction member.

In the first aspect, the first slit may include a plurality of first extending portions that extend in straight lines along the first surface and intersect with each other at a first point, the second slit may include a plurality of second extending portions that extend in straight lines along the second surface and intersect with each other at a second point, and the plurality of first extending portions and the plurality of second extending portions may be disposed alternately. In this case, the valve body for the medical device may appropriate suppress formation of the gap between the base and the introduction member.

In the first aspect, the plurality of first extending portions may be four first extending portions that extend radially from the first point and an angle between each of the first extending portions may be 90 degrees, the plurality of second extending portions may be four second extending portions that extend radially from the second point and an angle between each of the second extending portions may be 90 degrees, and when the slits are seen from a direction in which the first surface and the second surface oppose each other, positions of the first point and the second point may be aligned, and the four first extending portions and the four second extending portions may be disposed at equal angles. In this case, the valve body for the medical device may improve the operability when the user moves the introduction member from the first surface side toward the second surface side, while appropriately suppressing the formation of the gap between the base and the introduction member.

In the first aspect, the second length may be longer than the first length. In this case, the valve body for the medical device may cause an elastic force of a section in which the second slit is provided in the base to be larger than an elastic force of a section in which the first slit is provided. Thus, the base has a strong close fit with the introduction member on the second surface side on which the second slit having the relatively large elastic force is provided. As a result, the valve body for the medical device may even more appropriately suppress the blood inside the living organism from flowing from the gap between the base and the introduction member. The close fit between the base and the introduction member is weaker on the first surface side on which the first slit having the relatively small elastic force is provided. Thus, the valve body for the medical device may further improve the operability when the user moves the introduction member from the first surface side toward the second surface side.

In the first aspect, a part of the first slit and a part of the second slit may overlap in a direction in which the first surface and the second surface oppose each other. In this case, the valve body for the medical device may appropriately connect the first slit and the second slit at the connecting section.

A medical device according to a second aspect of the present invention is characterized by including: the valve body for the medical device according to the first aspect, a main body portion through which an introduction member is able to be inserted and which includes an insertion hole extending from a base end side toward a leading end side, and a support portion that supports the valve body for the medical device, the support portion supporting the valve body for the medical device, at a position intersecting the insertion hole, in a state in which the first surface is oriented toward the base end side and the second surface is oriented toward the leading end side, and supporting the valve body for the medical device by applying a force to the valve body for the medical device, wherein the base of the valve body for the medical device deforms in a convex shape toward the leading end side in accordance with the force from the support portion, the gap of the first slit becomes smaller, in accordance with the deformation of the base in the convex shape, than the gap of the first slit in the single state, and the gap of the second slit becomes larger, in accordance with the deformation of the base in the convex shape, than the gap of the second slit in the single state.

The medical device supports the valve body for the medical device in a state in which the base of the valve body for the medical device is deformed in the convex shape toward the leading end side. The base is inclined to the leading end side from a peripheral portion toward a center portion. An inclination direction of the base matches a movement direction of the introduction member when the introduction member is introduced into the living organism. The gap of the second slit becomes relatively larger in accordance with the deformation of the base in the convex shape. Thus, the medical device may improve the operability when the user moves the introduction member into the living organism. As a result, the user may easily introduce the introduction member into the living organism using the medical device. The gap of the first slit becomes relatively smaller in accordance with the deformation of the base in the convex shape. Thus, in the valve body for the medical device, the medical device may appropriately suppress blood inside the living organism from flowing from a gap between the base and the introduction member when the introduction member is introduced into the living organism.

A method for manufacturing a valve body for a medical device according to a third aspect of the present invention is a method for manufacturing a valve body for a medical device that is configured to be assembled to the medical device used to introduce an introduction member into a living organism. The method is characterized by including: a first step of manufacturing a base, the base including a first surface and a second surface that oppose each other, a first slit being formed in the base, the first slit extending from the first surface toward the second surface by a first length that is shorter than a base length, the base length being a length between the first surface and the second surface, and a second step of forming a second slit, by making cuts in the second surface using a blade, the second slit extending from the second surface toward the first surface of the base manufactured in the first step, by a second length that is shorter than the base length, the second slit having a gap smaller than a gap of the first slit in a single state in which the valve body is not assembled with the medical device, a part of the second slit being connected to the first slit.

According to the manufacturing method according to the third aspect of the present invention, for the valve body for the medical device, after manufacturing the base in which the first slit is formed in the first surface, the second slit is formed in the second surface of the base. The second slit is formed by making the cuts in the second surface using the blade. Thus, the second slit having the smaller gap than the first slit may be easily formed in the second surface of the base.

In the first aspect, the base in which the first slit may be formed is manufactured by a molding process using a die. In this case, the first slit having the larger gap than the second slit formed using the blade in the second step may be easily formed in the base.

### Brief description of the drawings

Fig. 1 is a side view of a Y connector 1.
Fig. 2 is a cross-sectional view of the Y connector 1.
Fig. 3 is a cross-sectional view of a main pipe member 2, a cap member 3, a hemostasis valve 5, a valve holding member 6, an opener 7, and a rotator 8.
Fig. 4 is a side view of the hemostasis valve 5.
Fig. 5 is a plan view of the hemostasis valve 5 as seen from a base end side.
Fig. 6 is a plan view of the hemostasis valve 5 as seen from a leading end side.
Fig. 7 is a cross-sectional view of the hemostasis valve 5 in an assembled state.
Fig. 8 is a flowchart showing a method for manufacturing the hemostasis valve 5.

### Description of Embodiments

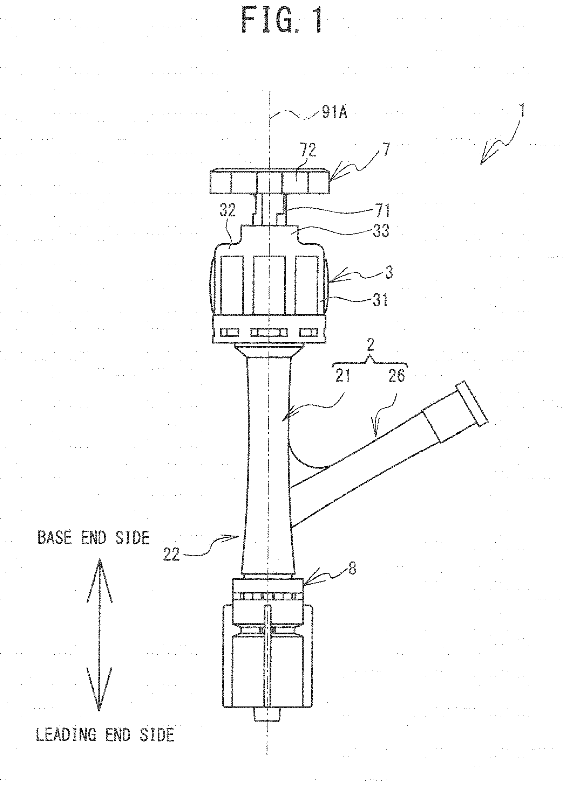

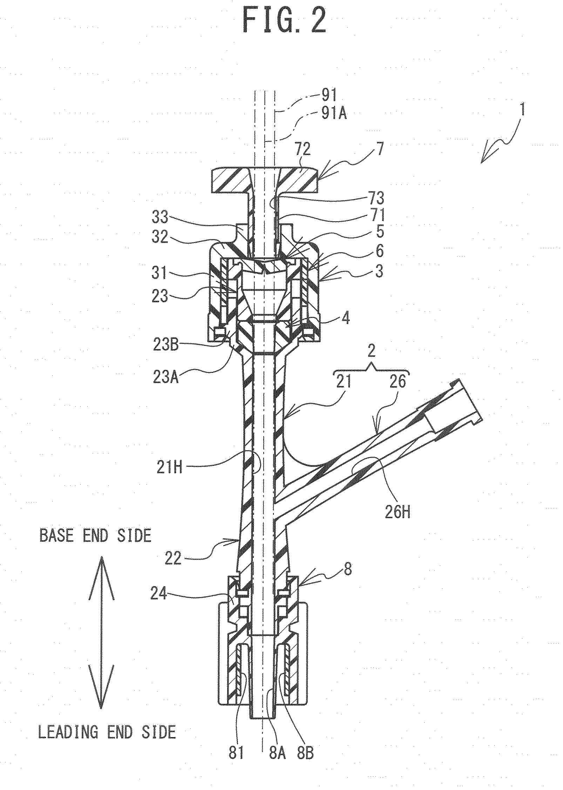

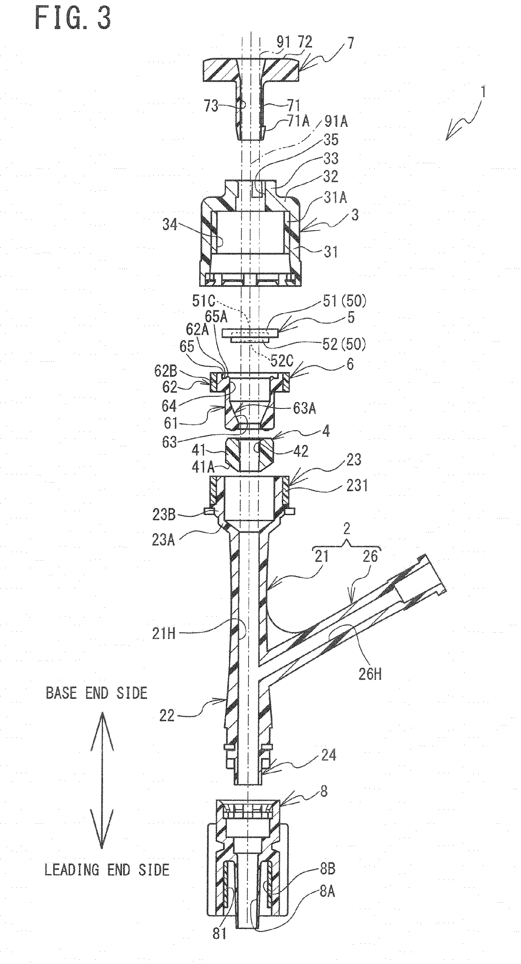

A Y connector 1 that is an embodiment of the present invention will be explained with reference to the drawings. The up-down direction in Fig. 1 is an "extending direction." The upper side in Fig. 1 is referred to as a base end side, and the lower side is referred to as a leading end side. Main structural elements of the Y connector 1 are a main pipe member 2, a cap member 3, a fixing valve 4 (refer to Fig. 2 and Fig. 3), a hemostasis valve 5 (refer to Fig. 2 and Fig. 3), a valve holding member 6 (refer to Fig. 2 and Fig. 3), an opener 7, and a rotator 8.

### Main pipe member 2

The main pipe member 2 is substantially Y-shaped. The main pipe member 2 is formed of a synthetic resin material, such as polycarbonate. The main pipe member 2 includes a main branch 21 and sub branch 26. The main branch 21 extends in a straight line shape along the extending direction. The main branch 21 is tubular shaped with a circular cross-sectional shape. As shown in Fig. 2 and Fig. 3, the main branch 21 includes a first section 22, a second section 23, a third section 24, and a main hole 21H.

A cross section of the first section 22 has substantially the same outer diameter over the extending direction. The second section 23 extends from a base end portion of the first section 22 toward the base end side. As shown in Fig. 3, the second section 23 has a tapered portion 23A and an expanded diameter portion 23B. The tapered portion 23A extends in a tapered shape toward the outside, from the base end portion of the first section 22. The expanded diameter portion 23B extends toward the base end side from a base end portion of the tapered portion 23A. A thread groove 231 is formed in the outer peripheral surface of the expanded diameter portion 23B. The second section 23 houses the fixing valve 4 that will be described later. The third section 24 extends toward the leading end side from a leading end portion of the first section 22. An outer diameter of a cross section of the third section 24 is smaller than that of the first section 22.

The main hole 21H is a through hole that extends along the extending direction. Of the main hole 21H, a diameter of a section provided in the first section 22 and in the third section 24 is the same. Of the main hole 21H, a diameter of a section provided in the second section 23 is larger than the diameter of the section provided in the first section 22 and in the third section 24. More specifically, the main hole 21H becomes larger toward the base end side at the tapered portion 23A of the second section 23. Of the main hole 21H, a diameter of a section provided in the expanded diameter portion 23B of the second section 23 is substantially twice that of the diameter of the section provided in the first section 22.

The sub branch 26 is continuous to a section of the main branch 21 that is further to the leading end side than a substantial center of the first section 22 in the extending direction. The sub branch 26 extends from a connection portion with the main branch 21, in a direction that is inclined to the base end side with respect to a direction orthogonal to the extending direction. The sub branch 26 extends in a straight line shape. The sub branch 26 is a tubular shaped member that has a circular cross-sectional shape. The sub branch 26 has a sub hole 26H. The main hole 21H of the main branch 21 and the sub hole 26H of the sub branch 26 are communicated with each other.

### Cap member 3

The cap member 3 is connected to the second section 23 of the main pipe member 2. As shown in Fig. 2, the cap member 3 houses the hemostasis valve 5 and the valve holding member 6 that will be described later. The cap member 3 has a bottomed cylindrical shape. The cap member 3 is formed of a transparent synthetic resin. As shown in Fig. 3, the cap member 3 includes a peripheral wall portion 31, a bottom portion 32, and a protruding portion 33.

The peripheral wall portion 31 is a cylindrically-shaped member that has a circular cross-sectional shape. The bottom portion 32 covers a base end portion of the peripheral wall portion 31. The protruding portion 33 protrudes from a center of the bottom portion 32 toward the base end side. A cap hole 34 is formed in a section surrounded by an inner peripheral surface of the peripheral wall portion 31. The protruding portion 33 has a cap hole 35 that extends along the extending direction. The cap holes 34 and 35 are communicated with each other. A thread ridge 31A is formed on the inner peripheral surface of the peripheral wall portion 31.

The cap member 3 is assembled with the main pipe member 2 by the thread ridge 31A being screwed into the thread groove 231 of the main branch 21. In a state in which the cap member 3 is assembled with the main pipe member 2, centers of each of the cap holes 34 and 35, and the main hole 21H are disposed on the same axis line (hereinafter referred to as an "axis line 91A").

### Valve holding member 6

As shown in Fig. 2, the valve holding member 6 is housed in the cap member 3. The valve holding member 6 houses the hemostasis valve 5 to be described later. The valve holding member 6 has a circular cylindrical shape. The valve holding member 6 is formed of a transparent synthetic resin. As shown in Fig. 3, the valve holding member 6 includes a first section 61, and a second section 62. The first section 61 and the second section 62 are cylindrically-shaped and have a circular cross-sectional shape. The second section 62 is disposed on the base end side of the first section 61.

The first section 61 has a holding hole 63. The holding hole 63 penetrates the first section 61 in the extending direction. A taper 63A, which is oriented such that a diameter thereof becomes larger toward the base end side, is formed on the leading end side of the holding hole 63. The second section 62 includes holding holes 64 and 65, and a thread groove 62B. The holding holes 64 and 65 penetrate the second section 62 in the extending direction. The holding hole 64 is provided on the leading end side of the second section 62. A leading end portion of the holding hole 64 is communicated with the holding hole 63 of the first section 61. A diameter of the holding hole 64 is larger than the cap hole 35 of the cap member 3. The holding hole 65 is provided on the base end side of the second section 62. A leading end portion of the holding hole 65 is communicated with the holding hole 64. A diameter of the holding hole 65 is larger than the diameter of the holding hole 64.

A level difference 62A is formed in a communication section of the holding holes 64 and 65. The level difference 62A is a flat surface joining the holding holes 64 and 65. The flat surface of the level difference 62A is oriented toward the base end side. The hemostasis valve 5, to be described later, is fitted into the holding holes 64, and 65, and the level difference 62A. A protruding portion 65A is provided on a section furthest to the inside of the level difference 62A, namely, a section of the level difference 62A that is connected to the holding hole 64. The protruding portion 65A protrudes from the level difference 62A toward the base end side. A shape of the protruding portion 65A is a ring shape when seen from the extending direction. A base end portion of the protruding portion 65A is curved. The protruding portion 65A does not protrude further to the base end side than a base end portion of the second section 62 of the valve holding member 6. The thread groove 62B is provided on the outer peripheral surface of the second section 62.

The valve holding member 6 is assembled with the cap member 3 by thread ridge 31A of the cap member 3 being screwed into the thread groove 62B. In a state in which the valve holding member 6 is assembled with the cap member 3, centers of the cap holes 34 and 35, and the holding holes 63, 64, and 65 are respectively disposed on the axis line 91A. After the valve holding member 6 has been assembled with the cap member 3, the main pipe member 2 and the cap member 3 are assembled by the thread ridge 31A being screwed into the thread groove 231 of the main branch 21. In a state in which the cap member 3 is assembled with the main pipe member 2, the first section 61 of the valve holding member 6 assembled with the cap member 3 is disposed on the inside of the expanded diameter portion 23B of the second section 23 of the main pipe member 2.

### Fixing valve 4

As shown in Fig. 2, the fixing valve 4 is housed in the second section 23 of the main pipe member 2. The fixing valve 4 has a circular cylindrical shape. The fixing valve 4 is formed of an elastic synthetic resin. As shown in Fig. 3, the fixing valve 4 includes a main body portion 41. The main body portion 41 is a columnar shape. A taper 41A, which is oriented such that a diameter thereof becomes larger toward the base end side, is formed on an outer peripheral surface of the leading end side of the main body portion 41. The main body portion 41 has a fixing hole 42. The fixing holes 42 penetrates the main body portion 41 in the extending direction. A diameter of the fixing hole 42 is substantially the same as the diameter of the main hole 21H of the main pipe member 2.

The fixing valve 4 is assembled with the main pipe member 2 and the cap member 3, by the cap member 3 being screwed onto the main pipe member 2 in a state in which the fixing valve 4 is housed inside the main pipe member 2. More specifically, the fixing valve 4 is assembled as described below. The thread groove 231 of the main pipe member 2, in which the fixing valve 4 is housed, and the thread ridge 31A of the cap member 3 are threadedly engaged with each other. The main pipe member 2 moves toward the base end side with respect to the cap member 3. The fixing valve 4 is clamped, from both sides in the extending direction, between the valve holding member 6 housed in the cap member 3, and the tapered portion 23A of the second section 23 of the main pipe member 2. The fixing valve 4 has a close fit with an inner peripheral surface of the second section 23 of the main pipe member 2. The fixing hole 42 of the fixing valve 4 is communicated with the main hole 21H of the main pipe member 2, and the holding hole 63 of the valve holding member 6.

### Hemostasis valve 5 (single state)

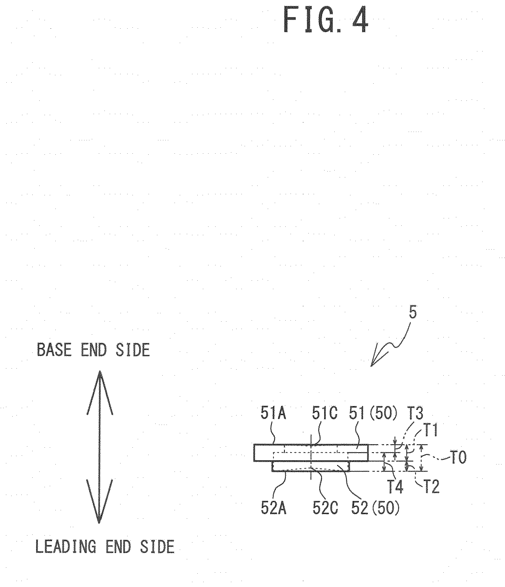

As shown in Fig. 2, the hemostasis valve 5 is held by the valve holding member 6. The hemostasis valve 5 in a state of not being assembled with the Y connector 1 (hereinafter referred to as a "single state") will be described with reference to Fig. 4 to Fig. 6. The hemostasis valve 5 has a circular plate shape. The hemostasis valve 5 is formed of an elastic material. Specific examples of the elastic material include synthetic rubbers such as silicon rubber, urethane rubber, butadiene rubber, and the like, natural rubber such as latex rubber, olefin-based elastomers (polystyrene elastomer, polypropylene elastomer, for example), polyamide elastomer, styrene-based elastomers, polyurethane, urethane-based elastomers, fluororesin-based elastomers, and so on. Of these, silicon rubber is particularly preferable.

The hemostasis valve 5 includes a first base 51 and a second base 52. The first base 51 and the second base 52 each have a circular plate shape. Each of surfaces of the first base 51 and the second base 52, respectively, are oriented in the extending direction. The first base 51 is disposed on the base end side of the second base 52. A diameter of the first base 51 is larger than a diameter of the second base 52. The diameter of the first base 51 is substantially the same as the diameter of the holding hole 65 of the valve holding member 6. The diameter of the second base 52 is substantially the same as the diameter of the holding hole 64 of the valve holding member 6. A surface on the leading end side of the first base 51 is in contact with a surface on the base end side of the second base 52. Of the first base 51, a surface on the opposite side to the surface that is in contact with the second base 52 is referred to as a "first surface 51A." Of the second base 52, a surface on the opposite side to the surface that is in contact with the first base 51 is referred to as a "second surface 52A." The first surface 51A and the second surface 52A are parallel to each other. Center portions of the first surface 51A and the second surface 52A are recessed. More specifically, the first surface 51A is inclined to the leading end side from a circumferential portion of the first base 51 toward a center point 51C. The second surface 52A is inclined to the base end side from a circumferential portion of the second base 52 toward a center point 52C. An inclination angle of the second surface 52A is larger than an inclination angle of the first surface 51A. Respective positions of the center point 51C of the first base 51 and the center point 52C of the second base 52 are aligned in a direction in which the first surface 51A and the second surface 52A oppose each other, namely, are aligned when seen from the extending direction.

A thickness of the circumferential portion of the first base 51 is referred to as a first base length, and is denoted by T1. A thickness of the peripheral portion of the second base 52 is referred to as a second base length, and is denoted by T2. The first base length T1 is larger than the second base length T2. The first base 51 and the second base 52 are collectively referred to as a "base 50." A thickness of the peripheral portion of the base 50 is referred to as a base length, and is denoted by T0. The base length T0 is equal to a value obtained by adding the first base length T1 and the second base length T2.

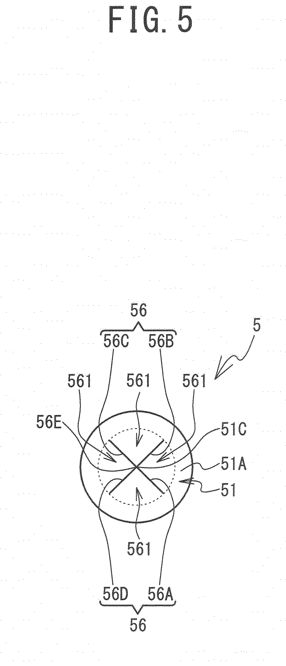

As shown in Fig. 5, the hemostasis valve 5 includes a first slit 56. The first slit 56 extends from the first surface 51A toward the second surface 52A side. A depth of the first slit 56 is referred to as a first slit length, and is denoted by T3 (refer to Fig. 4). The first slit length T3 is smaller than the first base length T1. The first slit 56 extends from the first surface 51A to the interior of the first base 51, and does not penetrate the first base 51 in the extending direction. A gap of the first slit 56 is 0.13 mm. The first slit 56 includes first extending portions 56A, 56B, 56C, and 56D. The first extending portions 56A to 56D extend in straight lines along the first surface 51A. The first extending portions 56A to 56D extend radially from the center point 51C toward the peripheral portion of the first base 51. The first extending portions 56A to 56D are disposed at equal intervals of 90 degrees. Of the base 50, 4 sections divided by the first extending portions 56A to 56D of the first slit 56 are each referred to as a "first valve portion 561."

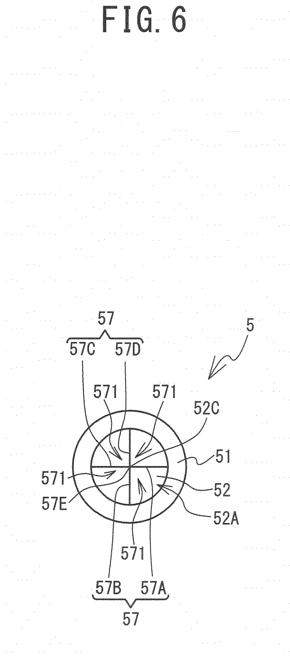

As shown in Fig. 6, the hemostasis valve 5 includes a second slit 57. The second slit 57 extends from the second surface 52A toward the first surface 51A side. A depth of the second slit 57 is referred to as a second slit length, and is denoted by T4 (refer to Fig. 4). The second slit length T4 is larger than the second base length T2, and is smaller than the base length T0. The second slit length T4 is larger than the first slit length T3. The second slit 57 extends from the second surface 52A, penetrates the second base 52, and extends further as far as the interior of the first base 51 on the base end side. A gap of the second slit is 0 mm. The gap of the second slit 57 is smaller than the gap of the first slit 56. The second slit 57 includes second extending portions 57A, 57B, 57C, and 57D. The second extending portions 57A to 57D extend in straight lines along the second surface 52A. The second extending portions 57A to 57D extend radially from the center point 52C toward the peripheral portion of the second base 52. The second extending portions 57A to 57D are disposed at equal intervals of 90 degrees. Of the base 50, 4 sections divided by the second extending portions 57A to 57D of the second slit 57 are each referred to as a "second valve portion 571."

Hereinafter, of the first slit 56, a section corresponding to the center point 51C is referred to as a "connecting section 56E." Of the second slit 57, a section corresponding to the center point 52C is referred to as a "connecting section 57E." The first slit 56 and the second slit 57 are connected at the connecting sections 56E and 57E. In other words, the first slit 56 and the second slit 57 penetrate the base 50 of the hemostasis valve 5, in the extending direction, at the connecting sections 56E and 57E.

As shown in Fig. 5 and Fig. 6, the first extending portions 56A to 56D of the first slit 56 and the second extending portions 57A to 57D of the second slit 57 are disposed alternately as seen from the extending direction. When seen from the base end side, the second extending portion 57A of the second slit 57 is disposed in a position obtained by rotating the first extending portion 56A of the first slit 56 by 45 degrees in the counterclockwise direction. Similarly, as seen from the base end side, the second extending portions 57B to 57D of the second slit 57 are disposed in positions obtained by rotating the first extending portions 56B to 56D of the first slit 56 by 45 degrees in the counterclockwise direction. As a result, the first extending portions 56A to 56D and the second extending portions 57A to 57D extend radially toward the peripheral portion of the base 50 from the center points 51C and 52C. When seen from the extending direction, the first extending portions 56A to 56D and the second extending portions 57A to 57D are disposed at equal intervals of 45 degrees. When seen from the base end side, the first extending portions 56A to 56D and the second extending portions 57A to 57D are disposed in the order of 56A, 57A, 56B, 57B, 56C, 57C, 56D, and 57D, in the counterclockwise direction.

As shown in Fig. 4, a value obtained by adding the first slit length T3 of the first slit 56 and the second slit length T4 of the second slit 57 is slightly larger than the base length T0 of the base 50. In other words, a part of a leading end portion of the first slit 56 overlaps, in the extending direction, with a part of a base end portion of the second slit 57.

### Hemostasis valve 5 (assembled state)

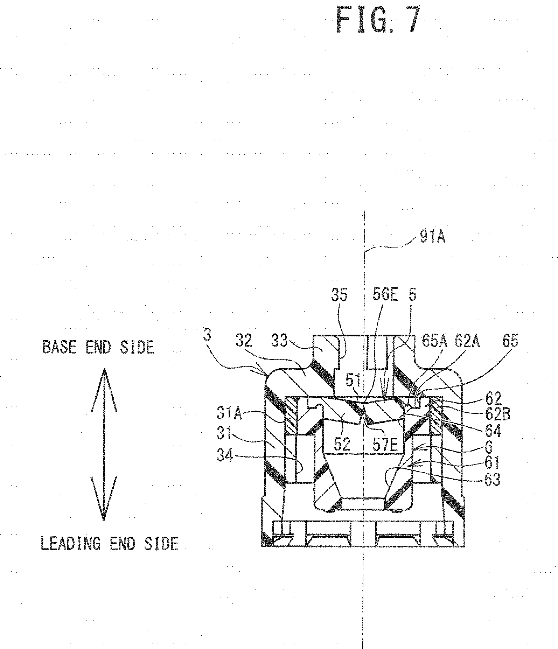

As shown in Fig. 7, the hemostasis valve 5 is assembled with the cap member 3 and the valve holding member 6. Hereinafter, the state of being assembled with the cap member 3 and the valve holding member 6 is referred to as an "assembled state." The hemostasis valve 5 is assembled with the cap member 3 and the valve holding member 6 in the following manner.

The hemostasis valve 5 is held inside the holding holes 64 and 65 of the valve holding member 6. The first base 51 of the hemostasis valve 5 is fitted into the inside of the holding hole 65 that is on the base end side. The diameter of the first base 51 is substantially the same as the diameter of the holding hole 65, and thus, the first base 51 has a close fit with the holding hole 65, from the inside. The second base 52 of the hemostasis valve 5 is fitted into the inside of the holding hole 64 that is on the leading end side. The diameter of the second base 52 is substantially the same as the diameter of the holding hole 64, and thus, the second base 52 has a close fit with the holding hole 64, from the inside. The protruding portion 65A of the valve holding member 6 comes into contact, from the leading end side, with a section, of the leading end side surface of the first base 51 of the hemostasis valve 5, which is in the vicinity of the second base 52. The section of the hemostasis valve 5 that comes into contact with the protruding portion 65A is recessed to the base end side.

The valve holding member 6 is screwed into the cap member 3. In accordance with the thread ridge 31A of the cap member 3 and the thread groove 62B of the valve holding member 6 being threadedly engaged with each other, the valve holding member 6 moves to the base end side with respect to the cap member 3. The base end portion of the second section 62 of the valve holding member 6 has a close fit with the bottom portion 32 of the cap member 3, from the leading end side. The first base 51 of the hemostasis valve 5 is clamped, from both sides in the extending direction, between the level difference 62A of the second section 62 of the valve holding member 6 and the bottom portion 32 of the cap member 3. The section of the hemostasis valve 5 that comes into contact with the protruding portion 65A of the valve holding member 6 is further recessed to the base end side. The hemostasis valve 5 is in the assembled state.

In the assembled state, the first base 51 of the hemostasis valve 5 is compressed by being clamped from both sides in the extending direction. Expansion of the first base 51 outward in the radial direction is restricted by the holding hole 65 of the valve holding member 6. A force that resists a force with which the first base 51 tries to expand outward in the radial direction is received, from the holding hole 65, as a force in a direction from the peripheral portion of the first base 51 toward the center portion. The center points 51C and 52C (refer to Fig. 4 to Fig. 6) of the hemostasis valve 5 try to warp to the base end side or the leading end side. The holding hole 64 of the valve holding member 6 is larger than the diameter of the cap hole 35 of the cap member 3. Thus, the center points 51C and 52C of the hemostasis valve 5 warp toward the leading end side corresponding to the side of the holding hole 64 of the valve holding member 6, and do not warp toward the base end side. The hemostasis valve 5 deforms in a convex shape toward the center points 51C and 52C from the peripheral portion of the base 50. The first surface 51A and the second surface 52A of the hemostasis valve 5 become inclined to the leading end side toward the center points 51C and 52C from the peripheral portion of the base 50.

In accordance with the deformation of the hemostasis valve 5, the gap of the first slit 56 (refer to Fig. 5) closes. A size of the gap of the first slit 56 in the assembled state is smaller than a size of the gap of the first slit 56 in the single state. On the other hand, the gap of the second slit 57 (refer to Fig. 6) opens. A size of the gap of the second slit 57 in the assembled state is larger than a size of the gap of the second slit 57 in the single state.

The movement of the hemostasis valve 5 is restricted by the protruding portion 65A coming into contact with the hemostasis valve 5 from the leading end side. When the first base 51 of the hemostasis valve 5 is clamped from both sides in the extending direction, the protruding portion 65A suppresses the hemostasis valve 5 from falling into the holding hole 64. The base end portion of the protruding portion 65A is curved, and thus, even when the protruding portion 65A comes into contact with the hemostasis valve 5, the hemostasis valve 5 is not damaged by the protruding portion 65A.

### Opener 7

As shown in Fig. 2 and Fig. 3, the opener 7 includes a tubular portion 71 and a flange portion 72. The tubular portion 71 has a circular cylindrical shape. The tubular portion 71 extends in the extending direction. An outer diameter of the tubular portion 71 is slightly smaller than the cap hole 35 of the cap member 3. The tubular portion 71 is inserted into the cap hole 35, toward the leading end side, from a base end portion of the protruding portion 33 of the cap member 3. The tubular portion 71 is held such that the tubular portion 71 can move in the extending direction with respect to the cap member 3. A protrusion 71A is provided on a leading end portion of the tubular portion 71. The protrusion 71A inhibits the tubular portion 71 from slipping out from the cap member 3. In a state in which the tubular portion 71 has moved furthermost to the base end side with respect to the cap member 3, the leading end portion of the tubular portion 71 is separated, to the base end side, from the first surface 51A of the hemostasis valve 5.

The flange portion 72 is provided on a base end portion of the tubular portion 71. The flange portion 72 has a circular plate shape. An outer diameter of the flange portion 72 is substantially the same as an outer diameter of the peripheral wall portion 31 of the cap member 3. When the tubular portion 71 has moved to the leading end side with respect to the cap member 3, the flange portion 72 comes into contact with the base end portion of the protruding portion 33 of the cap member 3. As will be described in more detail later, when the opener 7 has moved to the leading end side, the leading end portion of the tubular portion 71 opens the connecting sections 56E and 57E (refer to Fig. 5 and Fig. 6) of the hemostasis valve 5. The opener 7 includes an opener hole 73. The opener hole 73 penetrates centers of the tubular portion 71 and the flange portion 72 in the extending direction. A center of the opener hole 73 passes through the axis line 91 A.

### Rotator 8

The rotator 8 is attached, from the leading end side, to the third section 24 of the main branch 21 of the main pipe member 2. The rotator 8 is a member for connecting the Y connector 1 to a guiding catheter (not shown in the drawings). The rotator 8 includes an inner hole 8A and an outer hole 8B. The inner hole 8A is communicated with the main hole 21H of the main branch 21. The outer hole 8B is formed around the inner hole 8A. A groove 81 for mounting the guiding catheter is formed in an inner wall of the outer hole 8B.

When the main pipe member 2, the cap member 3, the fixing valve 4, the valve holding member 6, the opener 7, and the rotator 8 are assembled with each other, the main hole 21H, the cap holes 34 and 35, the holding holes 63, 64, and 65, the fixing hole 42, and the opener hole 73 are integrally communicated with each other. The main hole 21H, the cap holes 34 and 35, the holding holes 63, 64, and 65, the fixing hole 42, and the opener hole 73 are referred to as an "insertion hole 91."

### Method of use of Y connector 1

A method of use of the Y connector 1 will be explained. The introduction member, such as the catheter, the guide wire, or the like, is introduced into the insertion hole 91 by being inserted from the opener hole 73 of the opener 7. In the course of the introduction of the introduction member, a leading end portion of the introduction member comes into contact, from the base end side, with the first base 51 of the hemostasis valve 5. As described above, the hemostasis valve 5 is inclined to the leading end side toward the center points 51C and 52C from the peripheral portion of the base 50. In accordance with the introduction member moving from the base end side to the leading end side, the center points 51C and 52C of the hemostasis valve 5 further warp toward the leading end side. The hemostasis valve 5 opens the connecting sections 56E and 57E.

The introduction member passes through the opened connecting sections 56E and 57E. The four first valve portions 561 of the first base 51 and the four second valve portions 571 of the second base 52 have a close fit with side surfaces of the introduction member. In this way, the leakage of blood or the like from the leading end side toward the base end side of the hemostasis valve 5 via the insertion hole 91 is suppressed. The introduction member moves further to the leading end side and reaches the rotator 8. The introduction member is introduced into the guiding catheter mounted on the rotator 8, and is introduced into the human body via the guiding catheter.

A case will be exemplified in which the introduction member has reached a location of a treatment target inside the human body. In this case, an instrument, such as a solution supply device, is connected to the sub branch 26 of the main pipe member 2, and a fluid, such as a contrast medium or the like, is supplied. The supplied fluid enters into the guiding catheter via the main hole 21H of the main branch 21, and is administered to the location of the treatment target.

A case will be exemplified in which, in the course of introducing the introduction member, air is mixed in further to the leading end side than the hemostasis valve 5, or a complex operation of the introduction member is performed. In this case, the user performs an operation to move the opener 7 to the leading end side. The tubular portion 71 of the opener 7 is inserted into the connecting sections 56E and 57E of the hemostasis valve 5. The connecting sections 56E and 57E open. The four first valve portions 561 and the four second valve portions 571 of the hemostasis valve 5 are separated from the introduction member, and have a close fit with the side surfaces of the tubular portion 71 of the opener 7. In this state, the air mixed in further to the leading end side than the hemostasis valve 5 is released. Alternatively, since this is a state in which the movement of the introduction member is not obstructed by the hemostasis valve 5, the complex operation of the introduction member by the user becomes possible.

In the state in which the opener 7 has moved to the leading end side, the hemostasis valve 5 deforms in the convex shape to the leading end side. The opener 7 receives, from the hemostasis valve 5, the force in the direction toward the base end side. Thus, when the operation by the user to move the opener 7 to the leading end side is terminated, the opener 7 moves to the base end side as a result of the force received from the hemostasis valve 5, and returns to its original state.

A case will be exemplified in which the introduction member is used by being fixed to the Y connector 1. In this case, the user threadedly engages the cap member 3 with the main pipe member 2. The main pipe member 2 moves to the base end side with respect to the cap member 3. The fixing valve 4 is strongly clamped, from both sides in the extending direction, between the valve holding member 6 and the tapered portion 23A of the second section 23 of the main pipe member 2. The fixing hole 42 expands toward the inside in accordance with the main body portion 41 being compressed from both sides in the extending direction. The diameter of the fixing hole 42 contracts. The introduction member that is inserted into the fixing hole 42 is pressurized by the contraction of the diameter of the fixing hole 42, and cannot move in the extending direction with respect to the fixing valve 4. In this way, the user can fix the introduction member with respect to the Y connector 1.

### Manufacturing method of hemostasis valve 5

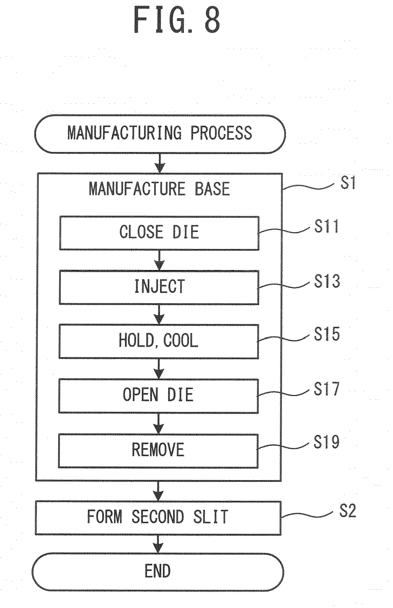

A manufacturing method of the hemostasis valve 5 will be explained with reference to Fig. 8. First, the base 50 is manufactured by a molding process using a die (S1). A specific example of a method of the molding process includes injection molding. Specifically, the process is performed as follows. As a result of a mold clamping process, the die is closed (S11). An elastic material that has been heated and melted is injected into the die by an injection process (S13). The elastic material is held until it is cooled by a pressure holding and cooling process (S15). The die is opened by a die opening process (S17). The base 50 is removed by a removal process (S19).

Apart from not including the second slit 57, the base 50 is the same as the base 50 of the hemostasis valve 5 shown in Fig. 4 to Fig. 6. The base 50 includes the first base 51 and the second base 52. The first slit 56 extends to a depth corresponding to the first slit length T3, from the first surface 51A of the first base 51 toward the second surface 52A of the second base 52. A gap of the first slit 56 is 0.13 mm. The first slit 56 includes the first extending portions 56A to 56D that extend radially from the center point 51C. At this point, the second slit 57 is not provided in the second surface 52A of the second base 52.

Next, as the second slit 57, the second extending portions 57A to 57D that extend radially from the center point 52C are formed in the base 50 that has been manufactured by the process at S1 (S2). The second extending portions 57A to 57D are formed by making cuts in the second surface 52A of the second base 52 using a blade. The second slit 57 extends to a depth corresponding to the second slit length T4, from the second surface 52A of the second base 52 toward the first surface 51A of the first base 51. Since the second slit 57 is formed by the blade, the gap of the second slit 57 is 0 mm. A part of a deepest section of the first slit 56 and a part of a deepest section of the second slit 57 overlap with each other in the opposing direction of the first surface 51A and the second surface 52A. The connecting sections 56E and 57E, which penetrate the base 50 in the opposing direction, are formed.

### Main operations and effects of present embodiment

As described above, in the single state, the hemostasis valve 5 includes the first slit 56 and the second slit 57 which have the different sized gaps. The introduction member is introduced into the living organism by the connecting sections 56E and 57E between the first slit 56 and the second slit 57 being moved from the first surface 51A side toward the second surface 52A side. The base 50 has a strong close fit with the introduction member on the second surface 52A side, in which the second slit 57 having the gap of 0 mm is provided. As a result, it is possible to appropriately suppress a flow of the blood inside the living organism from the second surface 52A side toward the first surface 51A side, from the gap between the four second valve portions 571 of the second base 52 and the introduction member. Further, on the first surface 51A side on which the first slit 56 having the gap of 1.3 mm is provided, the close fit between the four first valve portions 561 of the second base 52 and the introduction member is weaker than the close fit between the four second valve portions 571 of the second base 52 and the introduction member. Thus, the hemostasis valve 5 can improve operability when the user moves the introduction member from the first surface 51A side toward the second surface 52A side.

The first slit 56 includes the first extending portions 56A to 56D that extend radially from the center point 51C. The second slit 57 includes the second extending portions 57A to 57D that extend radially from the center point 52C. Respectively, the first extending portions 56A to 56D and the second extending portions 57A to 57D are disposed at the equal intervals of 90 degrees. The first extending portions 56A to 56D and the second extending portions 57A to 57D are disposed alternately when seen from the extending direction. The first extending portions 56A to 56D and the second extending portions 57A to 57D are disposed at the equal intervals of 45 degrees when seen from the extending direction. In this case, the four first valve portions 561 of the first base 51 that are divided by the first extending portions 56A to 56D, and the four second valve portions 571 of the second base 52 that are divided by the second extending portions 57A to 57D, come into contact with the introduction member alternately and at the equal intervals. In this case, the formation of a gap between the base 50 and the introduction member can be effectively suppressed, and the hemostasis valve 5 can appropriately suppress the blood inside the living organism from flowing from the gap.

If a number of the respective first extending portions and second extending portions is less than four, a contact area over which the first valve portions and the second valve portions come into contact with the introduction member becomes smaller. In this case, the gap between the base 50 and the introduction member occurs more easily, and there may be a case in which the blood easily leaks. On the other hand, if the number of the respective first extending portions and second extending portions becomes larger than four, the contact area over which the first valve portions and the second valve portions come into contact with the introduction member becomes larger. In this case, there may be a case in which the operability of the introduction member deteriorates. In contrast to this, the number of the respective first extending portions 56A to 56D and second extending portions 57A to 57D of the hemostasis valve 5 is four. In this way, the hemostasis valve 5 can maintain the operability when the user moves the introduction member from the first surface 51A side toward the second surface 52A side, while appropriately suppressing the formation of the gap between the base 50 and the introduction member.

The second slit length T4 that is the depth of the second slit 57 is longer than the first slit length T3 that is the depth of the first slit 56. In this case, in the base 50, an elastic force of a section in which the second slit 57 is provided can be made larger than an elastic force of a section in which the first slit 56 is provided. Thus, the base 50 has the strong close fit with the introduction member on the second surface 52A side on which the second slit 57 having the relatively large elastic force is provided. As a result, the hemostasis valve 5 can more appropriately suppress the blood inside the living organism from flowing from the gap between the base 50 and the introduction member. The hemostasis valve 5 can weaken the close fit between the base 50 and the introduction member on the first surface 51A side on which the first slit 56 having the relatively small elastic force is provided. Thus, the hemostasis valve 5 can further improve the operability when the user moves the introduction member from the first surface 51A side toward the second surface 52A side.

The value obtained by adding the first slit length T3 and the second slit length T4 is larger than the base length T0 of the base 50. Apart of each of the first slit 56 and the second slit 57 overlap in the extending direction. In this case, the hemostasis valve 5 can appropriately connect the first slit 56 and the second slit 57 at the connecting sections 56E and 57E, and thus, the base 50 can be penetrated via the first slit 56 and the second slit 57.

The Y connector 1 supports the hemostasis valve 5 in a state in which the base 50 of the hemostasis valve 5 is deformed in the convex shape toward the leading end side. The base 50 of the hemostasis valve 5 is inclined to the leading end side from the peripheral portion toward the center portion. An inclination direction of the base 50 matches a movement direction of the introduction member when the introduction member is introduced into the living organism. In accordance with the deformation of the base 50 in the convex shape, the size of the gap of the second slit 57 becomes larger. Thus, the close fit between the four second valve portions 571 and the introduction member becomes weaker, and the introduction member is easily moved. As a result, the user can easily introduce the introduction member into the living organism using the Y connector 1. Further, in accordance with the deformation of the base 50 in the convex shape, the gap of the first slit 56 becomes smaller. Thus, when the introduction member is introduced into the living organism using the Y connector 1, the hemostasis valve 5 can suppress, on the first surface 51A side, the blood in the living organism from flowing from the gap between the base 50 and the introduction member.

When the hemostasis valve 5 is manufactured, first, the base 50 is manufactured (S1) in which the first slit 56 is formed by injection molding in the first surface 51A, and the second slit 57 is not formed in the second surface 52A. After that, the second slit 57 is formed in the second surface 52A of the base 50. Note that the second slit 57 is formed by making the cuts in the second surface 52A using the blade (S2). By forming the second slit 57 by making the cuts using the blade, the hemostasis valve 5 can cause the gap of the second slit 57 to be 0 mm. As a result, by the above-described manufacturing method, the second slit 57 having the gap of 0 mm can be easily formed in the second surface 52A of the base 50. Note that the first slit 56 is formed by the injection molding. In this case, the first slit 56 can be formed at the same time as the manufacture of the base 50, and thus, the manufacturing process can be simplified.

### Modified examples

The present invention is not limited to the above-described embodiment, and various modifications are possible. The shape of the base 50 of the hemostasis valve 5 is not limited to the circular plate shape. For example, the base 50 may be a circular pillar, a square pillar, a truncated cone, a truncated pyramid, or the like. In the hemostasis valve 5, the second slit length T4 that is the depth of the second slit 57 is larger than the first slit length T3 that is the depth of the first slit 56. In contrast to this, the second slit length T4 and the first slit length T3 may be the same, or the first slit length T3 may be larger than the second slit length T4. In the single state, the gap of the second slit 57 is not limited to 0 mm, and may be a desired value so long as it is smaller than the gap of the first slit 56. The gap of the first slit 56 is not limited to 0.13 mm, and may be a desired value so long as it is larger than the second slit 57.

The first slit 56 includes the first extending portions 56A to 56D that extend radially from the center point 51C. The second slit 57 includes the second extending portions 57A to 57D that extend radially from the center point 52C. The number of the plurality of first extending portions, and the number of the plurality of second extending portions is not limited to four, and may be 2, 3 or 5 and above. The plurality of first extending portions and the plurality of second extending portions may be disposed at different intervals. At least some of the plurality of first extending portions and the plurality of second extending portions may be disposed in the same positions when seen from the extending direction. The length obtained by adding the first slit length T3 of the first slit 56 and the second slit length T4 of the second slit 57 may be the same as the base length T0 that is the thickness of the base 50. Another slit, another hole, or the like may be formed in addition to the first slit 56 and the second slit 57. The plurality of first extending portions 56A to 56D may extend radially from the center point 51C while curving along the first surface 51A. The plurality of second extending portions 57A to 57D may extend radially from the center point 52C while curving along the second surface 52A. A through hole may be provided at the center points 51C and 52C.

The shape of the hemostasis valve 5 in the assembled state may be the same as in the single state. In other words, in the state of the hemostasis valve 5 being held by the valve holding member 6, the center points 51C and 52C need not necessarily warp toward the leading end side. In the assembled state, the sizes of the gaps of the first slit 56 and the second slit 57 may be the same as the sizes of the gaps in the single state.

Under the conditions that the hemostasis valve 5 in the single state is held by the valve holding member 6, and the deformation to the outside of the hemostasis valve 5 in the assembled state is restricted by the valve holding member 6, the diameter of the first base 51 can be changed. Thus, the diameter of the first base 51 may be slightly smaller, or slightly larger, than the diameter of the holding hole 65 of the valve holding member 6.

In the hemostasis valve 5, first, the base 50 in which the second slit 57 is not formed is manufactured (S1), and next, the second slit 57 is formed by making the cuts using the blade (S2). In contrast to this, first, the base 50 may be formed in which the first slit 56 and the second slit 57 are not formed. Next, the first slit 56 and the second slit 57 may be formed by making cuts using a blade. The forming method of the base 50 is not limited to the molding process using the die. For example, the base 50 may be formed by a cutting out process.

In the assembled state, in accordance with the first base 51 being clamped and compressed from both sides in the extending direction, the hemostasis valve 5 comes into contact, from the inside, with the holding hole 65 of the valve holding member 6. The hemostasis valve 5 is subject to the force from the holding hole 65 in the direction from the peripheral portion toward the center portion. As a result of this force, the center points 51C and 52C of the hemostasis valve 5 warp toward the leading end side, and thus the hemostasis valve 5 deforms in the convex shape. In contrast to this, the valve holding member 6 may hold the hemostasis valve 5 in a state in which the force in the direction toward the leading end side with respect to the center points 51C and 52C of the hemostasis valve 5 is directly applied to the hemostasis valve 5. The center points 51C and 52C of the hemostasis valve 5 may warp toward the leading end side in accordance with the force received from the valve holding member 6. In this case, the hemostasis valve 5 need not necessarily come into contact with the holding hole 65 of the valve holding member 6.

### Other

The hemostasis valve 5 is an example of a "valve body for a medical device" of the present invention. The Y connector 1 is an example of the "medical device" of the present invention. The center point 51C corresponds to a "first point" of the present invention. The center point 52C corresponds to a "second point" of the present invention. The valve holding member 6 is an example of a "support portion" of the present invention. The main pipe member 2 is an example of a "main body portion" of the present invention.

## Claims

1. A valve body for a medical device that is configured to be assembled to the medical device used to introduce an introduction member into a living organism, the valve body for the medical device comprising:
a base including a first surface and a second surface that oppose each other; and
slits provided in the base,
wherein
the slits include a first slit and a second slit, the first slit extending from the first surface toward the second surface by a first length, the first length being shorter than a base length that is a length between the first surface and the second surface, the second slit extending from the second surface toward the first surface by a second length that is shorter than the base length, and at least a part of each of the first slit and the second slit are connected at a connecting section,
when the introduction member is introduced into the living organism, the introduction member passes through the connecting section and moves from the first surface side toward the second surface side, and
in a single state in which the valve body is not assembled with the medical device, a gap of the second slit is smaller than a gap of the first slit.

2. The valve body for the medical device according to claim 1, wherein
in the single state, the gap of the second slit is 0.

3. The valve body for the medical device according to claim 1 or 2, wherein
the first slit includes a plurality of first extending portions that extend in straight lines along the first surface and intersect with each other at a first point,
the second slit includes a plurality of second extending portions that extend in straight lines along the second surface and intersect with each other at a second point, and
the plurality of first extending portions and the plurality of second extending portions are disposed alternately.

4. The valve body for the medical device according to claim 3, wherein
the plurality of first extending portions are four first extending portions that extend radially from the first point and an angle between each of the first extending portions is 90 degrees,
the plurality of second extending portions are four second extending portions that extend radially from the second point and an angle between each of the second extending portions is 90 degrees, and
when the slits are seen from a direction in which the first surface and the second surface oppose each other, positions of the first point and the second point are aligned, and the four first extending portions and the four second extending portions are disposed at equal angles.

5. The valve body for the medical device according to any one of claims 1 to 4, wherein
the second length is longer than the first length.

6. The valve body for the medical device according to any one of claims 1 to 5, wherein
a part of the first slit and a part of the second slit overlap in a direction in which the first surface and the second surface oppose each other.

7. A medical device comprising:
the valve body for the medical device according to any one of claims 1 to 6,
a main body portion through which an introduction member is able to be inserted and which includes an insertion hole extending from a base end side toward a leading end side, and
a support portion that supports the valve body for the medical device, the support portion supporting the valve body for the medical device, at a position intersecting the insertion hole, in a state in which the first surface is oriented toward the base end side and the second surface is oriented toward the leading end side, and supporting the valve body for the medical device by applying a force to the valve body for the medical device,
wherein
the base of the valve body for the medical device deforms in a convex shape toward the leading end side in accordance with the force from the support portion,
the gap of the first slit becomes smaller, in accordance with the deformation of the base in the convex shape, than the gap of the first slit in the single state, and
the gap of the second slit becomes larger, in accordance with the deformation of the base in the convex shape, than the gap of the second slit in the single state.

8. A method for manufacturing a valve body for a medical device that is configured to be assembled to the medical device used to introduce an introduction member into a living organism, the method comprising:
a first step of manufacturing a base, the base including a first surface and a second surface that oppose each other, a first slit being formed in the base, the first slit extending from the first surface toward the second surface by a first length that is shorter than a base length, the base length being a length between the first surface and the second surface, and
a second step of forming a second slit, by making cuts in the second surface using a blade, the second slit extending from the second surface toward the first surface of the base manufactured in the first step, by a second length that is shorter than the base length, the second slit having a gap smaller than a gap of the first slit in a single state in which the valve body is not assembled with the medical device, a part of the second slit being connected to the first slit.

9. The method for manufacturing the valve body for the medical device according to claim 8, wherein
the base in which the first slit is formed is manufactured by a molding process using a die.
